# EUROPÄISCHE PATENTSCHRIFT

(11) **EP 0 497 177 B1**
(45) Veröffentlichungstag und Bekanntmachung des Hinweises auf die Patenterteilung: **18.01.1995**
(21) Anmeldenummer: 92100838.9
(22) Anmeldetag: 20.01.1992
(51) Int. Cl.: A61K 9/14, A61K 31/375, A61K 31/44, A61K 31/51, A61K 31/525, A61K 9/20

(54) **Vitamin enthaltende Formulierungen und deren Herstellung**
Vitamin containing formulations and their production
Formulations contenant des vitamines et leur préparation

(30) Priorität: 28.01.1991 CH 250/91; 20.11.1991 CH 3402/91
(43) Veröffentlichungstag der Anmeldung: 05.08.1992
(73) Patentinhaber: F. HOFFMANN-LA ROCHE AG, 4002 Basel (CH)
(72) Erfinder: Caviezel, Gerold, CH-4142 Münchenstein (CH); Mertin, Frank, CH-4312 Magden (CH); Tritsch, Jean-Claude, F-68300 St-Louis (FR)
(74) Vertreter: Cottong, Norbert A.

(56) Entgegenhaltungen:
- EP-A- 0 390 435
- WO-A-85/01877
- US-A- 3 293 132
- US-A- 4 533 674

## Beschreibung

Die vorliegende Erfindung betrifft die Zubereitung einer freifliessenden, nahezu oder hundertprozentig reinen Form von Vitamin B₁, B₂, B₆ oder C oder von Ascorbinsäure-Natriumsalz oder -Calciumsalz, welche Form nachträglich und je nach Bedarf zu Tabletten oder anderen festen Verabreichungsformen direkt verpresst oder unter Verwendung von gewünschten Hilfsstoffen in gemischte Formulierungen übergeführt werden kann.

Bestrebungen sind schon lange im Gange, in kommerziellem Massstab reine, direkt verpressbare Formen von Vitamin C und weiteren Vitaminen herzustellen, allerdings ohne grossen Erfolg. Die meisten bisher kommerziell erhältlichen, direkt verpressbaren Vitaminformen enthalten mindestens 2% Hilfsstoffe, durch deren Anwesenheit u.a. die Fliessfähigkeit und Verpressbarkeit der Pulver und die Härte, Abriebfestigkeit, Zerfallzeit und die Stabilität gegen Verfärbung der verpressten Formen, z.B. Tabletten, einschliesslich Brausetabletten, Kerne usw., verbessert werden. Allerdings stellt z.B. die Nichtwasserlöslichkeit, und demzufolge die Sedimentation, gewisser Hilfsstoffe bei der Auflösung des Vitamins in Wasser einen grossen Nachteil dar.

Eine bekannte Methode zur Zubereitung von Vitaminpulvern, die zwangsläufig kleinere Mengen an Hilfsstoffen enthalten, besteht darin, dass man eine wässrige Aufschlämmung des Vitamins, z.B. Vitamin C, und der Hilfsstoffe, wie Kohlehydrate und filmbildende hydrophile, organische kolloidale Materialien, z.B. Gelatine, wasserlösliche Caseinderivate, wasserlösliche Harze oder wasserlösliche Cellulosederivate, sprühtrocknet. Das so entstandene Pulver ist aber auch dann nicht direkt verpressbar und muss noch mit einem Gleitmittel vermischt werden, bevor es zu einer direkt verpressbaren Form gelangt. Eine solche Methode ist beispielsweise in der US-Patentschrift Nr. 3.293.132 beschrieben.

Eine weitere bekannte Methode zur Zubereitung von direkt verpressbaren Vitaminpulvern ist aus der US-Patentschrift Nr. 3.396.226 bekannt geworden. Hier wird ein Vitamin C-Pulver zubereitet, indem man eine wässrige Aufschlämmung von Vitamin C und einem Bindemittel, z.B. mikrokristalliner Cellulose, in Gegenwart eines Absorptionsmittels, insbesondere Siliciumdioxid, sprühtrocknet. Auch in diesem Fall muss dem so zubereiteten Vitaminpulver ein Gleitmittel, z.B. Magnesiumstearat, zugefügt werden, bevor es zu Tabletten und dergleichen verpresst wird. Eine Verbesserung gegenüber dieser Methode ist in der PCT-Anmeldung Nr. PCT/US 84/01694 (Publikationsnr. WO 85/01877) beschrieben, bei der das Gleitmittel nicht nachträglich mit dem Vitaminpulver vermischt, sondern während des Sprühtrocknungsverfahrens zugemischt wird oder sich bereits in der wässrigen Aufschlämmung befindet.

Die obigen bekannten Methoden sind allerdings aufwendig, da Vor- und eventuell auch noch Nachmischungsstufen benötigt werden. Ferner führen sie unmittelbar nach dem Sprühtrocknen zu Vitaminpulvern, die zwangsläufig einen wesentlichen Anteil an Hilfsstoffen aufweisen. Die vorliegende Erfindung löst weitgehend diese Probleme und stellt eine Vereinfachung der bisher verwendeten Zubereitungsmethoden dar.

Gemäss der vorliegenden Erfindung ist das Verfahren zur Zubereitung von freifliessendem, direkt verpressbarem, möglichst reinem Vitamin B₁, Vitamin B₂, Vitamin B₆, Vitamin C (Ascorbinsäure), Ascorbinsäure-Natriumsalz oder Ascorbinsäure-Calciumsalz dadurch gekennzeichnet, dass man eine 5 bis 25%ige wässrige Lösung oder Suspension des entsprechenden Vitamins bzw. Ascorbinsäure-Natriumsalzes oder -Calciumsalzes sprühtrocknet.

Durch das erfindungsgemässe Verfahren wird die physikalische Form der Ausgangsware, d.h. des Vitamins B₁, B₂, B₆, C oder Ascorbinsäure-Natriumsalzes oder -Calciumsalzes, so verändert, dass das Produkt u.a. wesentlich bessere Presseigenschaften besitzt. Darüber hinaus wird das Produkt in nahezu reinem, wenn nicht hundertprozentig reinem, Zustand erhalten, d.h. die Verwendung von Hilfsstoffen im Sprühtrocknungsverfahren erübrigt sich. Der Reinheitsgrad beträgt mindestens 99,5%, in den meisten Fällen sogar 99,7 bis 100%. Da das Produkt direkt verpressbar ist, entfallen u.a. die herkömmliche aufwendige Granulatherstellung, Grob- und Feinsiebung, Zwischentrocknung und Vermischung, welche mit grossem Energie- und Zeitaufwand sowie Lösungsmittelproblemen behaftet sind. Das sind die Ziele und Ergebnisse der vorliegenden Erfindung.

Zur Durchführung des erfindungsgemässen Verfahrens wird eine wässrige Lösung oder Suspension der Ausgangsware entsprechender Konzentration in einen Sprühtrocknungsturm, zweckmässigerweise auf an sich bekannte Weise, eingesprüht. Zur Zerstäubung der Lösung bzw. Suspension bei deren Einleitung in den Turm eignen sich sowohl statische Einstoff- oder Zweistoffdüsen als auch Rotationsdüsen. Es erfolgt im Turm eine schnelle Verdampfung des Lösungsmittels bzw. Suspensionsmittels (Wasser), was durch die gleichzeitige Einleitung heisser Luft gefördert wird. Diese Luft kann im Gleich- oder Gegenstrom eingeleitet werden. Das so entstandene feine Pulver wird teils im unteren Bereich des Turms gesammelt (die "Turmware"), teils mit der Abluft in einen seitlich angeordneten Zyklon ausgezogen und dort an einem Filter gesammelt (die "Zyklonware"). Die Eigenschaften der Turmware und der Zyklonware können sich voneinander etwas unterscheiden, beide Waren sind jedoch gut verpressbar.

Die Konzentration der wässrigen Lösung oder Suspension der Ausgangsware beträgt im allgemeinen und je nach Löslichkeits- bzw. Suspendierbarkeitsgrenze 5 bis 25% (Gewichtsprozent), vorzugsweise 5 bis 12% und insbesondere ca. 10%. Zweckmässigerweise wird diese Lösung bzw. Suspension bei Raumtemperatur (etwa 15°C bis 25°C) in den Sprühtrocknungsturm eingeführt. Je nach Löslichkeit bzw. Suspendierbarkeit und Stabilität der Ausgangsware, gewünschter Konzentration der Lösung bzw. Suspension sowie technischen Eigenschaften der Sprühanlage kann aber die Lösung oder Suspension vor dem Einsprühen bis auf ca. 80°C erhitzt werden. Vorzugsweise beträgt die Temperatur der eingesprühten Lösung oder Suspension Raumtemperatur.

Die Temperatur der gleichzeitig eingeleiteten heissen Luft ("Zuluft") beträgt vorzugsweise 120°C bis 200°C, insbesondere ca. 130°C bis 150°C. Auch die Geschwindigkeit, bei der die Zuluft eingeleitet wird, beeinflusst die Qualität (Verpressbarkeit usw.) des Produktes. Die optimale Geschwindigkeit hängt von vielen Faktoren ab, wie beispielsweise der Einleitungsgeschwindigkeit der wässrigen Lösung oder Suspension der Ausgangsware, der sonstigen Natur der Zerstäubung (Grad der Vernebelung, Geometrie des Stroms usw.) und dem Volumen und der Geometrie des Sprühtrocknungsturms, und kann daher nicht allgemein quantifiziert werden. Der Fachmann wird sie von Fall zu Fall bestimmen können.

Bei Verwendung von rotierenden Zerstäubersystemen (Rotationsdüsen) spielt die Drehzahl der Düsen hinsichtlich der Qualität des Produktes eine Rolle. Wie im Falle der optimalen Einleitungsgeschwindigkeit der Zuluft (siehe oben) hängt diese Drehzahl jedoch von verschiedenen Faktoren ab. Auch hier wird sie der Fachmann von Fall zu Fall bestimmen können. Falls man zum Einsprühen der Lösung oder Suspension der Ausgangsware statische Einstoffdüsen (auch unter dem Namen Druckzerstäubungsdüsen bekannt), bei denen die Zerstäubung durch hydraulischen Druck erfolgt, verwendet, beträgt der Druck, unter dem die Lösung oder Suspension in den Sprühtrocknungsturm eingesprüht wird, geeigneterweise 10 bis 100 kg/cm² (9,8067 x 10⁵ bis 9,8067 x 10⁶ Pa). Im Falle der Verwendung von statischen Zweistoffdüsen, bei denen die Zerstäubung durch kinetische Energie erfolgt, beträgt dieser Druck geeigneterweise 2 bis 10 kg/cm² (1,9613 x 10⁵ bis 9,8067 x 10⁵ Pa). Mittels letzterer Düsen werden die Lösung bzw. Suspension und Sprühluft gleichzeitig in den Turm eingesprüht.

Sämtliche obenerwähnten Parameter, d.h. die verwendete Konzentration der Lösung bzw. Suspension im Bereich von 5 bis 25 Gewichtsprozent, deren Temperatur und Druck beim Einsprühen, die Temperatur der Zuluft, deren Einleitungsgeschwindigkeit, die Natur der Düsenvorrichtung, das Volumen und Geometrie des Sprühtrocknungsturms usw., beeinflussen die Qualität des erfindungsgemäss zubereiteten Vitaminpulvers und stehen in einem Abhängigkeitsverhältnis miteinander. Der Fachmann wird das Verhältnis optimal bestimmen können.

Die obengenannten Vitamine können im erfindungsgemässen Verfahren sowohl als solche als auch in Form ihrer üblichen Salze verwendet werden. Falls Vitamin B₁ im erfindungsgemässen Verfahren verwendet wird, wird dies sehr bevorzugt in Form seines Hydrochlorid- oder Nitratsalzes eingesetzt. Vitamin B₂ wird vorzugsweise als Riboflavin selbst oder als Riboflavinphosphatnatriumsalz, und Vitamin B₆ als Pyridoxin-Hydrochlorid oder Pyridoxal-Phosphat, eingesetzt. Die entsprechenden Formen werden unter Verwendung des erfindungsgemässen Verfahrens in freifliessende, direkt verpressbare, möglichst reine Form umgewandelt.

Das erfindungsgemässe Verfahren eignet sich allerdings vorzugsweise zur Zubereitung von freifliessendem, direkt verpressbarem und möglichst reinem Pyridoxin-Hydrochlorid, Vitamin C oder Ascorbinsäure-Natriumsalz, insbesondere Vitamin C.

Nach erfolgter Sprühtrocknung kann das Produkt, egal ob Turm- oder Zyklonware, weiterverarbeitet werden, insbesondere auf konventionelle Weise direkt zu Tabletten oder anderen festen Verabreichungsformen verpresst werden. Dies kann mit der Turmware, mit der Zyklonware oder mit einem Gemisch der beiden Produkte gewünschten Verhältnisses geschehen. Gewünschtenfalls kann man aber als Vorstufe auch beliebig kleinere Mengen eines oder mehrerer Hilfsstoffe, z.B. ein Gleitmittel, zumischen, um den anschliessend hergestellten Tabletten und dergleichen besondere Eigenschaften zu verleihen, z.B. noch höhere Härte, Abriebfestigkeit, Zerfallzeit, Stabilität usw. Da das Produkt des erfindungsgemässen Verfahrens ein nahezu oder hundertprozentig reines Vitaminpulver ist, kann die endgültige Zusammensetzung nach Zusatz von Hilfsstoffen beliebig bestimmt werden, um die gewünschten Eigenschaften viel leichter als bisher zu bestimmen. Ebenfalls können verschiedene erfindungsgemäss zubereitete Produkte miteinander vermischt werden, um zu gemischten Vitaminformulierungen zu gelangen.

Die vorliegende Erfindung betrifft auch noch eine erfindungsgemäss zubereitete freifliessende, nahezu oder hundertprozentig reine Form von Vitamin B₁, B₂, B₆ oder C oder von Ascorbinsäure-Natriumsalz oder -Calciumsalz sowie Tabletten und andere feste Verabreichungsformen, die aus einer solchen freifliessenden Form des obigen Vitamins bzw. Ascorbinsäure-Natriumsalzes oder -Calciumsalzes, oder aus einem Gemisch eines oder mehrerer davon, durch Verpressung gebildet werden.

Die nachfolgenden Beispiele dienen zur näheren Erläuterung der Erfindung:

### Beispiel 1

Eine wässrige Lösung von reinem Vitamin C mit einer Konzentration von 5, 10 oder 15% (Gewichtsprozent) wird bei Raumtemperatur mittels Rotationsdüsen (ca. 23.000 Umdrehungen/Min.) in einen Sprühtrocknungsturm eingesprüht. Gleichzeitig wird in den Turm heisse Luft bei 130-170°C und einer Geschwindigkeit von 1000-2000 m³/Stunde eingeleitet.

Das resultierende sprühgetrocknete Vitamin C-Pulver wird teils im unteren Bereich des Turms, teils im seitlich angeordneten Zyklon des Turms gesammelt. Die entsprechende Turm- und Zyklonware wird entfernt und deren Feuchtigkeit (Wassergehalt) und Partikelgrösse auf konventionelle Weise gemessen. Auch deren Stabilität gegen Färbung wird untersucht.

Dann wird jede Ware mit modifizierter Stärke (Sta-Rx 1500) sowie Gleitmittel (Magnesiumstearat) vermischt, um jeweils zu einer Zusammensetzung zu gelangen, die aus 90,90, 7,6 resp. 1,5 Gewichtsprozent der drei Komponenten besteht. Die Zusammensetzung wird anschliessend unter einer Presskraft von 2500 KP (Kilopounds = 1,134 x 10⁵ kg) zu Vitamin C 500 mg Tabletten verpresst, und diese werden auf Härte, Abriebfestigkeit und Zerfallzeit (in Wasser bei 37°C) geprüft. Auch die Standardabweichung des Tablettengewichtes wird gemessen.

Sowohl die Turmware als auch die Zyklonware zeigen eine äusserst niedrige Feuchtigkeit (weniger als 0,5%igen Wassergehalt) und eine gute Stabilität gegen Verfärbung. Die weiteren Ergebnisse der verschiedenen obenerwähnten Messungen und Untersuchungen sind in der nachstehenden Tabelle 1 zusammengefasst:

**Tabelle 1**

| a) 5%ige Vitamin C-Lösung | Turmware | Zyklonware |
|---|---|---|
| sprühgetrocknetes Pulver:- | | |
| Partikelgrösseverteilung | 92-220 µm | 32-124 µm |
| Durchschnittliche Partikelgrösse | 129 µm | 66 µm |

| Vitamin C 500 mg Tabletten:- | | |
|---|---|---|
| Härte | 124 N | 121 N |
| Abriebfestigkeit | 0,72 % | 0,54 % |
| Standardabweichung des Tablettengewichts | 0,38 % | 1,05 % |
| Zerfallzeit (in Wasser bei 37°C) | 55 Sek. | 45 Sek. |

| b) 10%ige Vitamin C-Lösung | | |
|---|---|---|
| sprühgetrocknetes Pulver:- | | |
| Partikelgrösseverteilung | 100-297 µm | 30-142 µm |
| Durchschnittliche Partikelgrösse | 160 µm | 75 µm |

| Vitamin C 500 mg Tabletten:- | | |
|---|---|---|
| Härte | 120 N | 119 N |
| Abriebfestigkeit | 1,76 % | 0,18 % |
| Standardabweichung des Tablettengewichts | 0,58 % | 1,49 % |
| Zerfallzeit (in Wasser bei 37°C) | 65 Sek. | 55 Sek. |

| c) 15%ige Vitamin C-Lösung | | |
|---|---|---|
| sprühgetrocknetes Pulver:- | | |
| Partikelgrösseverteilung | 120-291 µm | 38-167 µm |
| Durchschnittliche Partikelgrösse | 170 µm | 88 µm |

| Vitamin C 500 mg Tabletten:- | | |
|---|---|---|
| Härte | 68 N | 110 N |
| Standardabweichung des Tablettengewichts | 0,66 % | 0,51 % |
| Zerfallzeit (in Wasser bei 37°C) | 50 Sek. | 60 Sek. |

### Beispiel 2

Eine wässrige Lösung von reinem Ascorbinsäure-Natriumsalz mit einer Konzentration von 10% (Gewichtsprozent) wird bei Raumtemperatur mittels Rotationsdüsen (ca. 23.000 Umdrehungen/Min.) in einen Sprühtrocknungsturm eingesprüht. Gleichzeitig wird in den Turm heisse Luft bei 130°C (Zuluft) und einer Geschwindigkeit von 2.000 m³/Stunde eingeleitet. Die Ablufttemperatur beträgt 105°C.

Das resultierende sprühgetrocknete Ascorbinsäure-Natriumsalz-Pulver wird teils im unteren Bereich des Turms, teils im seitlich angeordneten Zyklon des Turms gesammelt. Die entsprechende Turm- und Zyklonware wird entfernt.

Jede Ware, sowie Ascorbinsäure-Natriumsalz-Granulate USP (eine Ware der United States Pharmacopoeia-Qualität) wird einzeln mit Ascorbinsäure 98% DC, Zucker DC (White Di-Pac®), Maisstärke, Aspartame® und Magnesiumstearat vermischt, um jeweils zu einer Zusammensetzung zu gelangen, wie diese in der nachfolgenden Tabelle 2a angegeben ist:

**Tabelle 2a**

| Zusammensetzung: | Standart | Turmware | Zyklonware |
|---|---|---|---|
| Ascorbinsäure 98% DC (1) | 130,1 mg | 130,1 mg | 130,1 mg |
| Ascorbinsäure-Natriumsalz-Granulate USP | 143,5 mg | --------- | -------- |
| Ascorbinsäure-Natriumsalz 100% DC Turm (1) | | 143,5 mg | -------- |
| Ascorbinsäure-Natriumsalz 100% DC Zyklon (1) | | | 143,5 mg |
| Zucker DC (1) [White Di-Pac® (2)] | 295,0 mg | 295,0 mg | 295,0 mg |
| Maisstärke | 43,9 mg | 43,9 mg | 43,9 mg |
| Aspartame® (3) | 1,5 mg | 1,5 mg | 1,5 mg |
| Magnesiumstearat | 6,0 mg | 6,0 mg | 6,0 mg |
| | 620,0 mg | 620,0 mg | 620,0 mg |

| | | | |
|---|---|---|---|
| (1) DC bedeutet "direct compressible" (direkt verpressbar) | | | |
| (2) White Di-Pac® ist ein im Handel erhältlicher Zucker (Amstar, USA) | | | |
| (3) Aspartame® ist L-Aspartyl-L-phenylalaninmethylester, ein von Ajinomoto und Searle entwickelter potentieller Süssstoff mit 140 mal stärkerer Süsskraft als Saccharose. | | | |

Dann wird jede Zusammensetzung unter einer Presskraft von 2000 KP zu Ascorbinsäure-Natriumsalz-Tabletten verpresst, und diese werden auf Härte und Abriebfestigkeit getestet. Auch die Standardabweichung des Tablettengewichts wird jeweils gemessen. Die Ergebnisse sind in der nachstehenden Tabelle 2b zusammengefasst:

**Tabelle 2b**

| Ascorbinsäure-Natriumsalz-Tabletten: | Standart | Turmware | Zyklonware |
|---|---|---|---|
| Härte | 83N | 135N | 156N |
| Abriebfestigkeit | --- | 0,80% | 0,48% |
| Standardabweichung des Tablettengewichts | 0,24% | 0,41% | 0,35% |

### Beispiel 3

Unter Verwendung von Vitamin B₆-Hydrochlorid anstelle von Ascorbinsäure-Natriumsalz werden analog dem in Beispiel 2 beschriebenen Verfahren folgende Standart-, Turmware- und Zyklonware-Zusammensetzungen bereitgestellt:

**Tabelle 3a**

| Zusammensetzung: | Standart | Turmware | Zyklonware |
|---|---|---|---|
| Vitamin B₆-HCl DC 98% (1) | 443,48 mg | -------- | --------- |
| Vitamin B₆-HCl 100% DC Turm (1) | -------- | 443,48 mg | --------- |
| Vitamin B₆-HCl 100 DC Zyklon (1) | -------- | --------- | 443,48 mg |
| Avicel®PH 102(4) | 51,30 mg | 51,30 mg | 51,30 mg |
| Magnesiumstearat | 5,22 mg | 5,22 mg | 5,22 mg |
| | 500,0 mg | 500,0 mg | 500,0 mg |

| | | | |
|---|---|---|---|
| (1) siehe Beispiel 2 | | | |
| (4) Avicel® ist mikrokristalline Cellulose (FMC Corp., USA). Die "PH"-Produkte werden als Füll-, Binde-, Fliess-, Dispersions- und Trägerstoff, insbesondere bei der Tablettierung in der pharmazeutischen Industrie, verwendet. | | | |

Dann wird jede Zusammensetzung unter einer Presskraft von 1000 KP zu Vitamin B₆-Hydrochlorid-Tabletten verpresst, und diese werden auf Härte und Abriebfestigkeit getestet. Die Ergebnisse sind in der nachstehenden Tabelle 3b zusammengefasst:

**Tabelle 3b**

| Vitamin B₆-Hydrochlorid-Tabletten: | Standart | Turmware | Zyklonware |
|---|---|---|---|
| Härte | 56N | 91N | 82N |
| Abriebfestigkeit | --- | 0,80% | 0,25% |

## Patentansprüche

1. Verfahren zur Zubereitung von freifliessendem, direkt verpressbarem, möglichst reinem Vitamin B₁, Vitamin B₂, Vitamin B₆, Vitamin C, Ascorbinsäure-Natriumsalz oder Ascorbinsäure-Calciumsalz, dadurch gekennzeichnet, dass man eine 5 bis 25%ige wässrige Lösung oder Suspension des entsprechenden Vitamins bzw. Ascorbinsäure-Natriumsalzes oder -Calciumsalzes sprühtrocknet.

2. Verfahren nach Anspruch 1, worin der Reinheitsgrad des zubereiteten Vitamins bzw. Ascorbinsäure-Natriumsalzes oder -Calciumsalzes 99,7 bis 100% beträgt.

3. Verfahren nach Anspruch 1 oder 2, worin die Konzentration des Vitamins bzw. Ascorbinsäure-Natriumsalzes oder -Calciumsalzes in der Wässrigen Lösung oder Suspension 5 bis 12% beträgt.

4. Verfahren nach einem der Ansprüche 1 bis 3, worin die wässrige Lösung oder Suspension bei Raumtemperatur in einen Sprühtrocknungsturm, in den gleichzeitig heisse Luft eingeleitet wird, eingesprüht wird.

5. Verfahren nach Anspruch 4, worin die Temperatur der heissen Luft 120°C bis 200°C beträgt.

6. Verfahren nach einem der Ansprüche 1 bis 5, worin freifliessendes, direkt verpressbares und möglichst reines Vitamin C zubereitet wird.

7. Verfahren nach einem der Ansprüche 1 bis 5, worin freifliessendes, direkt verpressbares und möglichst reines Vitamin B₁-Hydrochlorid, Vitamin B₁-Nitrat, Riboflavinphosphatnatriumsalz, Pyridoxin-Hydrochlorid oder Pyridoxal-Phosphat zubereitet wird.

8. Freifliessendes, nahezu oder hundertprozentig reines Vitamin B₁, B₂, B₆ oder C oder Ascorbinsäure-Natriumsalz oder -Calciumsalz, dadurch gekennzeichnet, dass es nach einem der Ansprüche 1 bis 7 zubereitet wurde.

9. Tabletten und andere feste Verabreichungsformen aus Vitamin B₁, B₂, B₆ oder C oder Ascorbinsäure-Natriumsalz oder -Calciumsalz, oder aus einem Gemisch eines oder mehrerer dieser Substanzen, dadurch gekennzeichnet, dass sie aus dem entsprechenden freifliessenden, nahezu oder hundertprozentig reinen Vitamin bzw. Salz gemäss Anspruch 8 gebildet wurden.

## Claims

1. A process for the production of free-flowing, directly compressible, as pure as possible vitamin B₁, vitamin B₂, vitamin B₆, vitamin C, sodium ascorbate or calcium ascorbate, characterized by spray drying a 5 to 25% aqueous solution or suspension of the corresponding vitamin or sodium or calcium ascorbate.

2. A process according to claim 1, wherein the degree of purity of the vitamin or sodium or calcium ascorbate produced is 99.7 to 100%.

3. A process according to claim 1 or 2, wherein the concentration of the vitamin or sodium or calcium ascorbate in the aqueous solution or suspension is 5 to 12%.

4. A process according to any one of claims 1 to 3, wherein the aqueous solution or suspension is sprayed at room temperature into a spray drying tower into which hot air is simultaneously introduced.

5. A process according to claim 4, wherein the temperature of the hot air is 120°C to 200°C.

6. A process according to any one of claims 1 to 5, wherein free-flowing, directly compressible and as pure as possible vitamin C is produced.

7. A process according to any one of claims 1 to 5, wherein free-flowing, directly compressible and as pure as possible vitamin B₁ hydrochloride, vitamin B₁ nitrate, riboflavin phosphate sodium salt, pyridoxine hydrochloride or pyridoxal phosphate is produced.

8. Free-flowing, almost pure or one hundred percent pure vitamin B₁, B₂, B₆ or C or sodium ascorbate or calcium ascorbate, characterized in that it has been produced according to any one of claims 1 to 7.

9. Tablets and other solid doasage forms of vitamin B₁, B₂, B₆ or C or sodium ascorbate or calcium ascorbate or of a mixture of one or more of these substances, characterized in that they have been formed from the corresponding free-flowing, almost pure or one hundred percent pure vitamin or salt in accordance with claim 8.

## Revendications

1. Procédé pour la préparation des vitamines B₁, B₂, B₆ et C, du sel de sodium ou de calcium de l'acide ascorbique, s'écoulant librement, directement compressibles et aussi purs que possible, caractérisé en ce que l'on sèche par pulvérisation une solution ou une suspension aqueuse contenant 5 à 25% de la vitamine correspondante ou du sel de sodium ou de calcium de l'acide ascorbique.

2. Procédé selon la revendication 1, dans lequel le degré de pureté de la vitamine ou du sel de sodium ou de calcium de l'acide ascorbique préparé est compris entre 99,7 et 100%.

3. Procédé selon la revendication 1 ou 2, dans lequel la concentration de la vitamine ou du sel de sodium ou de calcium de l'acide ascorbique dans la solution ou dans la suspension aqueuse est comprise entre 5 et 12%.

4. Procédé selon l'une des revendications 1 à 3, dans lequel la solution ou la suspension aqueuse est pulvérisée à la température ambiante dans une tour de séchage par pulvérisation, dans laquelle on introduit en même temps de l'air chaud.

5. Procédé selon la revendication 4, dans lequel la température de l'air chaud est comprise entre 120°C et 200°C.

6. Procédé selon l'une des revendications 1 à 5, dans lequel on prépare de la vitamine C s'écoulant librement, directement compressible et aussi pure que possible.

7. Procédé selon l'une des revendications 1 à 5, dans lequel on prépare le chlorhydrate de la vitamine B₁, le nitrate de la vitamine B₁, le sel de sodium du phosphate de riboflavine, le chlorhydrate de pyridoxine ou le phosphate de pyridoxal, s'écoulant librement, directement compressible et aussi purs que possible.

8. Les vitamines B₁, B₂, B₆ ou C ou le sel de sodium ou de calcium de l'acide ascorbique, s'écoulant librement, d'une pureté de cent pour cent ou proche de cent pour cent, caractérisés en ce qu'ils sont préparés selon l'une des revendications 1 à 7.

9. Comprimés et autres formes d'administration solides de vitamines B₁, B₂, B₆ ou C ou de sel de sodium ou de calcium de l'acide ascorbique ou d'un mélange d'une ou plusieurs de ces substances, caractérisés en ce qu'ils sont formés à partir de la vitamine ou du sel correspondant, s'écoulant librement, d'une pureté de cent pour cent ou proche de cent pour cent, selon la revendication 8.
